# EUROPEAN PATENT APPLICATION

(11) **EP 3 550 503 A1**
(43) Date of publication of application: **09.10.2019**
(21) Application number: 17875789.4
(22) Date of filing: 26.07.2017
(51) Int. Cl.: G06Q 50/22, G06Q 10/04

(54) **INFORMATION PROCESSING METHOD, INFORMATION PROCESSING DEVICE, AND INFORMATION PROCESSING TERMINAL**

(30) Priority: 29.11.2016 JP 2016231791
(71) Applicant: Sony Mobile Communications Inc., Shinagawa-ku Tokyo 140-0002 (JP)
(72) Inventor: HIROBE, Keisuke, Tokyo 140-0002 (JP)
(74) Representative: D Young & Co LLP
(86) International application number: PCT/JP2017/026962
(87) International publication number: WO 2018/100797

(57) **Abstract**

[Object] To provide an information processing method, an information processing apparatus, and an information processing terminal that can enhance care prevention.

[Solution] Provided is an information processing method including: acquiring, by an acquisition unit, measurement results of physical fitness measurement and a cognition function, life information, and a social participation score; learning, by a learning unit, correlation between a physical fitness value and a cognition function value that are based on the measurement results, and life information and a social participation score; and predicting, by a prediction unit, future physical fitness value and cognition function value of a user using the learned correlation on the basis of life information and a social participation score of the user.

## Description

### Technical Field

The present disclosure relates to an information processing method, an information processing apparatus, and an information processing terminal.

### Background Art

The existing physical fitness measurement is conducted about once a year, and in the measurement, recording is manually performed using a timer and a tape measure, and muscle strength or the like of a specific region is measured using a dedicated tool.

Regarding a physical fitness measurement apparatus, for example, Patent Literature 1 described below discloses a physical fitness determination apparatus including an output unit that outputs an instruction of an exercise to be performed by a user, an exercise detection unit that detects an exercise of the user, and a physical fitness measurement unit that calculates an exercise matching degree being a matching degree of a detection result of the exercise detection unit with respect to an exercise instruction, and measures fitness for performance on the basis of the exercise matching degree.

In addition, such physical fitness measurement is not limited to children and young people, and the measurement of physical fitness and cognition functions of elderly people has also become important. Regarding physical fitness measurement of elderly people, for example, Patent Literature 2 described below discloses a physical fitness measurement method that is safe and appropriate for elderly people, and can estimate physical fitness levels corresponding to the measurements of "repeated side step" and "vertical jump" only by performing the measurement of "stairs climb up-down time", by measuring the stairs climb up-down time, the repeated side step, and the vertical jump, and obtaining correlation between the stairs climb up-down time, the repeated side step, and the vertical jump.

In addition, Patent Literature 3 described below discloses a cognition function evaluation system including an apparatus that performs measurement of a physical function of a measurement subject, and an evaluation apparatus that processes numerical data obtained by the apparatus that performs the measurement of a physical function, and evaluates a cognition function of the measurement subject.

### Citation List

### Patent Literature

Patent Literature 1: JP 2004-73272A
Patent Literature 2: JP 2000-300710A
Patent Literature 3: JP 2011-115362A

### Disclosure of Invention

### Technical Problem

Because the above-described existing physical fitness measurements involve a lot of troubles and man-hours, and in addition, require a large space, such physical fitness measurements could be conducted only about once a year, and it has been difficult to frequently conduct such physical fitness measurements. Nevertheless, conditions of physical fitness and cognition functions of elderly people fluctuate from day to day, and in some cases, accurate numerical values could not be obtained by annual measurement. In this case, there is concern that care prevention cannot be sufficiently performed.

The care prevention is defined as delaying a person getting into a state requiring care as far as possible, and even if a person is in the state requiring care, achieving improvement in such a manner that the state does not become further worse. For the care prevention, while prevention of daily declines in physical fitness and cognition functions of elderly people is important, the above-described physical fitness measurement conducted about once a year is insufficient, and in addition, an appropriate advice in daily life has not been offered.

In view of the foregoing, the present disclosure proposes an information processing method, an information processing apparatus, and an information processing terminal that can enhance care prevention.

### Solution to Problem

According to the present disclosure, there is provided an information processing method including: acquiring, by an acquisition unit, measurement results of physical fitness measurement and a cognition function, life information, and a social participation score; learning, by a learning unit, correlation between a physical fitness value and a cognition function value that are based on the measurement results, and life information and a social participation score; and predicting, by a prediction unit, future physical fitness value and cognition function value of a user using the learned correlation on the basis of life information and a social participation score of the user.

Moreover, according to the present disclosure, there is provided an information processing apparatus including: an acquisition unit configured to acquire measurement results of physical fitness measurement and a cognition function, life information, and a social participation score; a learning unit configured to learn correlation between a physical fitness value and a cognition function value that are based on the measurement results, and life information and a social participation score; and a prediction unit configured to predict future physical fitness value and cognition function value of a user using the learned correlation on the basis of life information and a social participation score of the user.

Moreover, according to the present disclosure, there is provided an information processing terminal including: a receiving unit configured to receive detected data from a sensor device attached to a leg portion of a user; and a measurement unit configured to measure physical fitness of the user on the basis of the received detected data.

### Advantageous Effects of Invention

As described above, according to the present disclosure, it becomes possible to enhance care prevention.

Note that the effects described above are not necessarily limitative. With or in the place of the above effects, there may be achieved any one of the effects described in this specification or other effects that may be grasped from this specification.

### Brief Description of Drawings

FIG. 1 is a diagram describing an overview of an information processing system according to an embodiment of the present disclosure.
FIG. 2 is a diagram describing a care prevention cycle according to the present embodiment.
FIG. 3 is a block diagram illustrating an example of a configuration of a user terminal according to the present embodiment.
FIG. 4 is a block diagram illustrating an example of a configuration of a server according to the present embodiment.
FIG. 5 is a diagram illustrating a screen display example in a phonation duration time measurement test being an example of a measurement item according to the present embodiment.
FIG. 6 is a diagram describing measurement of a phonation duration time according to the present embodiment.
FIG. 7 is a diagram illustrating a screen display example in an agility test being an example of a measurement item according to the present embodiment.
FIG. 8 is a diagram illustrating a screen display example in a tap test being an example of a measurement item according to the present embodiment.
FIG. 9 is a diagram illustrating a screen display example in a step test being an example of a measurement item according to the present embodiment.
FIG. 10 is a diagram illustrating a screen display example in a leg raise test being an example of a measurement item according to the present embodiment.
FIG. 11 is a diagram illustrating a screen display example in a squat test being an example of a measurement item according to the present embodiment.
FIG. 12 is a diagram illustrating a screen display example in a memory test being an example of a measurement item according to the present embodiment.
FIG. 13 is a diagram illustrating a screen display example in an attention test being an example of a measurement item according to the present embodiment.
FIG. 14 is a sequence diagram illustrating a learning process according to the present embodiment.
FIG. 15 is a flowchart illustrating a prediction process according to the present embodiment.
FIG. 16 is a diagram describing social participation score calculation according to the present embodiment.
FIG. 17 is a diagram illustrating an example of a time-series analysis result of physical fitness and cognition function scores according to the present embodiment.
FIG. 18 is a diagram describing prediction of care necessity according to the present embodiment.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

In addition, the description will be given in the following order.
1. Overview of Information Processing System According to Embodiment of Present Disclosure
2. Configuration
   2-1. Configuration of User Terminal 1
   2-2. Configuration of Server 3
3. Measurement of Physical Fitness and Cognition Function
4. Operational Process
   4-1. Learning Process
   4-2. Prediction Process
5. Conclusion

### <<1. Overview of Information Processing System According to Embodiment of Present Disclosure>>

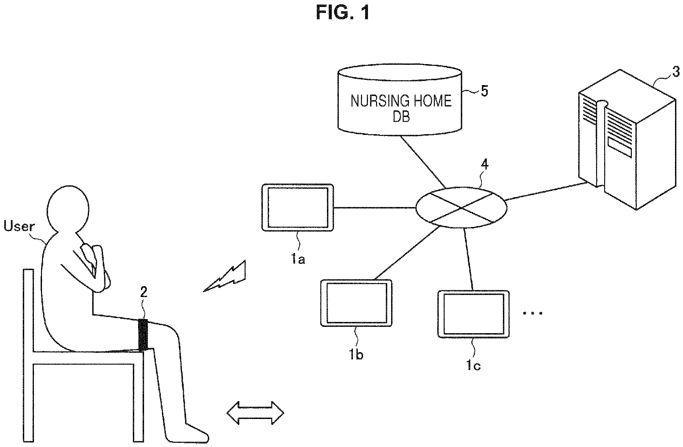

FIG. 1 is a diagram describing an overview of an information processing system according to an embodiment of the present disclosure. As illustrated in FIG. 1, the information processing system according to the present embodiment includes user terminals 1 (1a to 1c, and so on) that measure physical fitness and cognition functions of respective users (elderly people), and a server 3 that receives measurement results of the respective users from the user terminals 1, accumulates the measurement results, and performs analysis including machine learning.

The user terminal 1 (information processing terminal) is an information processing terminal such as a tablet terminal or a smartphone, and displays or voice-outputs a predetermined action instruction for physical fitness and cognition function measurement. In response to the action instruction, the user raises or lowers his/her legs in a state of being seated on a chair, or repeats standing up and sitting down, for example. These actions are detected by a sensor device 2 attached to a leg portion or the like of the user as illustrated in FIG. 1, and are transmitted to the user terminal 1 by wireless communication such as Bluetooth (registered trademark) or Wi-Fi (registered trademark), or wired communication. The user terminal 1 analyzes sensor data received from the sensor device 2, and performs automatic measurement (digitizing) and recording of physical fitness and a cognition function. In addition, the user terminal 1 transmits a measurement result to the server 3 via a network 4.

The sensor device 2 attached to the user includes an acceleration sensor, a gyro sensor, and the like, detects a motion of the user, and transmits the detected sensor data to the user terminal 1.

Aside from accumulating the measurement results of the respective users, the server 3 (information processing apparatus) continues machine learning using life information (diet, exercise, sleep, and basic information) and a social participation score of the user (elderly person) as parameters, and using physical fitness and cognition function scores as correct answer labels, and acquires correlation between the physical fitness and cognition function scores, and the life information and the social participation score. The server 3 can thereby calculate chronological changes in the physical fitness and cognition function scores on the basis of the life information and the social participation score of the user, predict future physical fitness value and cognition function value, and further predict whether or not the user is to require care in the future.

In a case where the user lives in a nursing home, the life information of the user can be acquired from a nursing home database (DB) 5 via the network 4. In addition, the social participation score is obtained by digitizing a social participation situation of the user such as visiting a specific location or participating in an event.

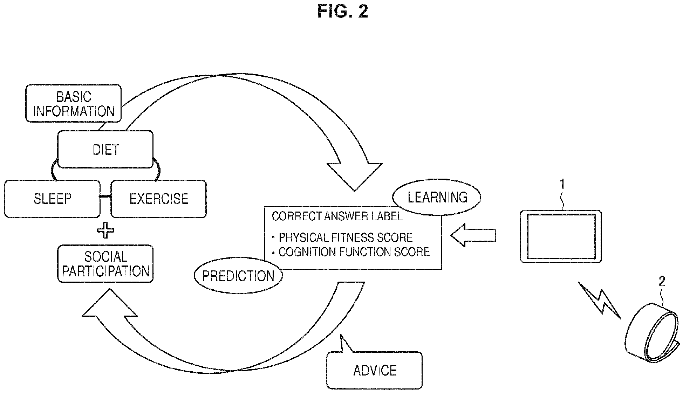

Then, in accordance with the prediction of future physical fitness value and cognition function value of the user, the server 3 generates a life situation improvement advice or an advice for prompting social participation, presents the generated advice to the user, prevents declines in physical fitness and a cognition function, and enables enhancement in care prevention. Here, a care prevention cycle according to the present embodiment will be described with reference to FIG. 2.

FIG. 2 is a diagram describing a care prevention cycle according to the present embodiment. As illustrated in FIG. 2, in the information processing system according to the present embodiment, on the basis of life information ("diet", "exercise", "sleep", and "basic information") obtained in daily life, and social participation information, time-series analysis of physical fitness and cognition function scores of the user is performed, and future physical fitness value and cognition function value of the user, and whether or not the user is to require care in the future are predicted. Then, an advice for care prevention that is related to a life situation and social participation is generated and presented to the user. In accordance with the advice, the user can improve the life situation or increase social participation for care prevention.

By these being repeated, care prevention of the user can be enhanced.

In addition, the time-series analysis of physical fitness and cognition function scores of the user is performed on the basis of a correlation rule of "physical fitness and a cognition function" and "life information and a social participation score" that is obtained by machine learning that uses life information and a social participation score as parameters, and uses measurement results of physical fitness and a cognition function that have been collected from the user terminal 1, as correct answer labels.

Note that, in the present embodiment, the "basic information" includes personal data (body height, body weight, gender, age, etc.), vital (body temperature, blood pressure, beat, SpO2, etc.), character, and the like. The personal data and the character may be pre-registered. The vital can be automatically acquired by a wearable device such as a SmartBand or SmartWatch that is worn by the user on a daily basis.

In addition, the "diet information" includes an intake timing, an amount (calorie), a water intake amount, and the like. In a case where the user lives in a nursing home, the diet information can be acquired from the nursing home DB 5. In addition, a diet intake timing can be recorded by a wearable device side by the user pressing a predetermined button of the wearable device at the time of intake, for example.

In addition, the "sleep information" includes sleeping hours, a sleep timing, depth of sleep, and the like. These types of information can also be automatically acquired by the wearable device.

In addition, the "exercise information" includes training, functional training, rehabilitation, the number of steps/an amount of activity, and the like. In a case where the user lives in a nursing home, these types of information can be acquired from the nursing home DB 5. In addition, "the number of steps/an amount of activity" can be automatically acquired by the wearable device worn by the user.

In addition, the "social participation information" includes the type of an event, a recreation, or the like that the user has participated in, and in addition, information regarding a location or the like that the user has visited, and a social participation score can be calculated by a system side on the basis of the social participation information. The type of an event, a recreation, or the like that the user has participated in can be acquired from the nursing home DB 5. In addition, the information regarding a location that the user has visited is acquired by a beacon receiving device (observer) installed at a predetermined location, for example. Specifically, for example, a wearable device worn by a user continuously transmits a beacon, and a beacon receiving device that has received the beacon extracts a user ID from the beacon and transmits the user ID to the server 3. The server 3 can thereby recognize the user that has visited a predetermined location (e.g. a conversation room, a dining room, a training room, etc. in a nursing home). In addition, the server 3 can also recognize a location that the user has visited, on the basis of absolute position information measured by the wearable device.

Note that the wearable device worn (or owned) by the user includes various types of sensors such as a biosensor, a position measurement unit (indoor/outdoor positioning), an acceleration sensor, and a gyro sensor, and a communication unit (Bluetooth, Wi-Fi, etc.), for example. In addition, the wearable device may also serve as the above-described sensor device 2 used in the measurement.

Hereinbefore, the information processing system according to an embodiment of the present disclosure has been described. Subsequently, specific configurations of the respective apparatuses included in the information processing system according to the present embodiment will be described with reference to the drawings.

### <<2. Configuration>>

### <2-1. Configuration of User Terminal 1>

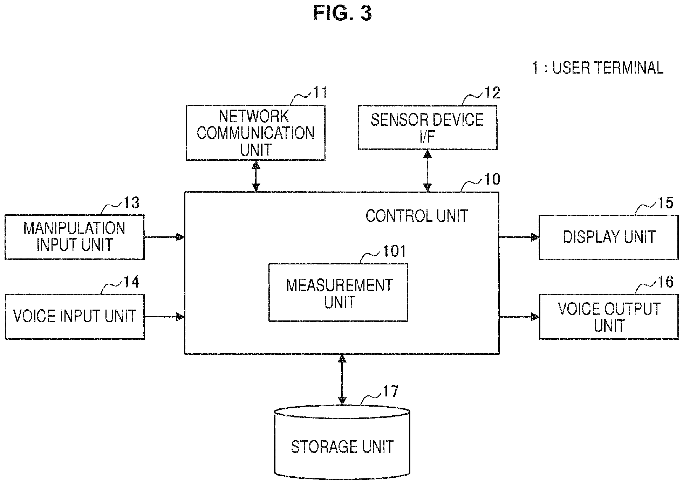

FIG. 3 is a block diagram illustrating an example of a configuration of the user terminal 1 according to the present embodiment. As illustrated in FIG. 3, the user terminal 1 includes a control unit 10, a network communication unit 11, a sensor device interface (I/F) 12, a manipulation input unit 13, a voice input unit 14, a display unit 15, a voice output unit 16, and a storage unit 17.

The control unit 10 functions as an arithmetic processing device and a control device, and controls the entire operations in the user terminal 1 in accordance with various programs. The control unit 10 is implemented by an electronic circuit such as a central processing unit (CPU) or a microprocessor, for example. In addition, the control unit 10 may include a read only memory (ROM) that stores programs to be used, calculation parameters, and the like, and a random access memory (RAM) that temporarily stores appropriately varying parameters and the like.

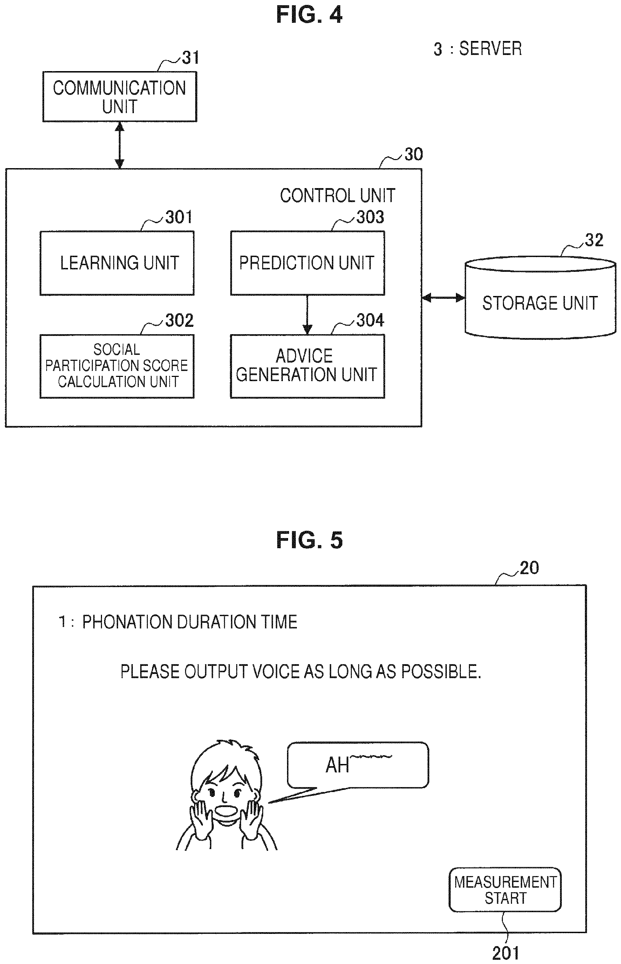

In addition, the control unit 10 according to the present embodiment also functions as a measurement unit 101. The measurement unit 101 controls an action instruction for instructing an action for measurement, to be output from the display unit 15 or the voice output unit 16, and performs measurement (digitizing) of physical fitness or a cognition function on the basis of an action, manipulation content, or phonation of the user that corresponds to the action instruction. The action of the user corresponds to detected data transmitted from the sensor device 2, for example, the manipulation content of the user can be input from the manipulation input unit 13, and in addition, the phonation of the user can be input by the voice input unit 14. Specific measurement content according to the present embodiment will be described later with reference to FIGS. 5 to 13.

The network communication unit 11 connects with the network 4 in a wired or wireless manner, and performs data transmission and reception with the server 3 on the network. The network communication unit 11 establishes communication connection with the network 4 by a wired/wireless local area network (LAN), Wi-Fi (registered trademark), a cellular communication network (Long Term Evolution (LTE), third-generation mobile communication method (3G)), or the like, for example.

The sensor device I/F 12 connects with the sensor device 2 in a wired or wireless manner, and receives detected data from the sensor device 2. For example, the sensor device I/F 12 establishes communication connection with the sensor device 2 by the Bluetooth, Wi-Fi, or the like.

The manipulation input unit 13 receives a manipulation instruction issued by the user, and outputs manipulation content thereof to the control unit 10. The manipulation input unit 13 may be a touch sensor, a pressure sensor, or a proximity sensor that is provided integrally with the display unit 15. Alternatively, the manipulation input unit 13 may be a physical configuration provided separately from the display unit 15, such as a button, a switch, and a lever.

The voice input unit 14 is implemented by a microphone, a microphone amplifier unit that performs amplification processing of a voice signal obtained by the microphone, and an A/D converter that performs digital conversion on the voice signal, and outputs the voice signal to the control unit 10.

The display unit 15 is a display device that outputs a manipulation screen, a menu screen, or the like. The display unit 15 may be a display device such as a liquid crystal display (LCD) or an organic electroluminescence (EL) display, for example. The display unit 15 according to the present embodiment displays an action instruction screen for instructing an action for measurement, in accordance with the control of the control unit 10.

The voice output unit 16 includes a speaker that reproduces the voice signal, and an amplifier circuit for the speaker. The voice output unit 16 according to the present embodiment may output action instruction voice for instructing an action for measurement, in accordance with the control of the control unit 10.

The storage unit 17 is implemented by a read only memory (ROM) that stores programs to be used in the processing of the control unit 10, calculation parameters, and the like, and a random access memory (RAM) that temporarily stores appropriately varying parameters and the like. In addition, the storage unit 17 according to the present embodiment may store the detected data transmitted from the sensor device 2, and a measurement result obtained by the measurement unit 101.

Hereinbefore, the configuration of the user terminal 1 according to the present embodiment has been specifically described. Note that the configuration of the user terminal 1 illustrated in FIG. 3 is an example, and the present embodiment is not limited to this.

### <2-2. Configuration of Server 3>

FIG. 4 is a block diagram illustrating an example of a configuration of the server 3 according to the present embodiment. As illustrated in FIG. 4, the server 3 (information processing apparatus) includes a control unit 30, a communication unit 31, and a storage unit 32.

### (Control Unit 30)

The control unit 30 functions as an arithmetic processing device and a control device, and controls the entire operations in the server 3 in accordance with various programs. The control unit 30 is implemented by an electronic circuit such as a central processing unit (CPU) or a microprocessor, for example. In addition, the control unit 30 may include a read only memory (ROM) that stores programs to be used, calculation parameters, and the like, and a random access memory (RAM) that temporarily stores appropriately varying parameters and the like.

In addition, the control unit 30 according to the present embodiment also functions as a learning unit 301, a social participation score calculation unit 302, a prediction unit 303, and an advice generation unit 304.

The learning unit 301 performs machine learning using life information (diet, exercise, sleep, and basic information) and a social participation score of the user (elderly person) as parameters, and using physical fitness and cognition function scores as correct answer labels, and acquires correlation between the physical fitness and cognition function scores, and the life information and the social participation score.

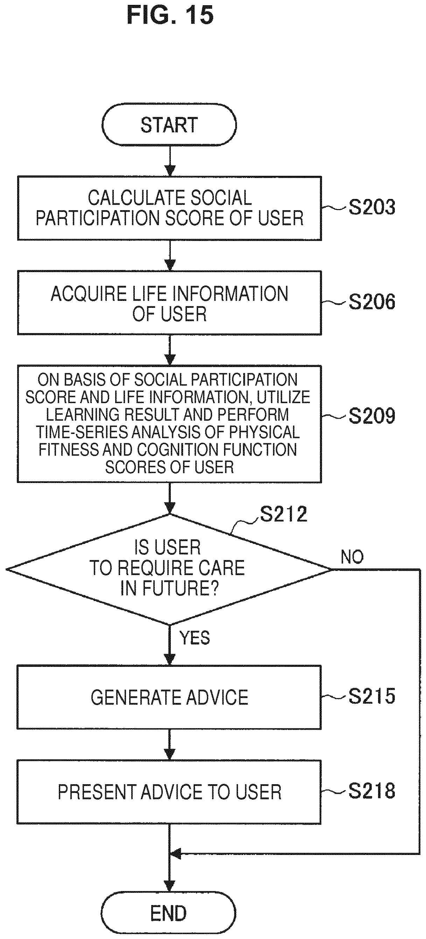

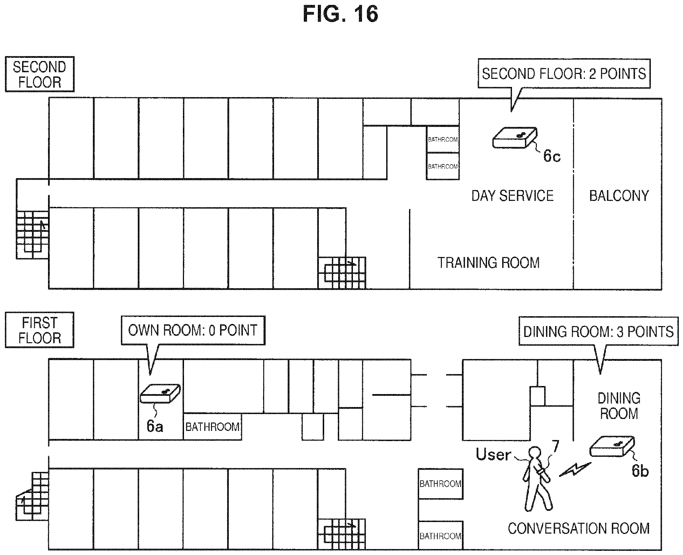

The social participation score calculation unit 302 digitizes and acquires a social participation score on the basis of social participation information of the user such as visiting a specific location or participating in an event. The details of calculation of the social participation score according to the present embodiment will be described later with reference to FIGS. 15 and 16.

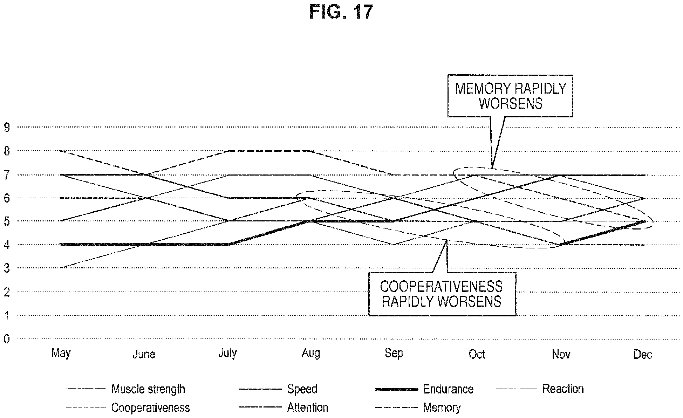

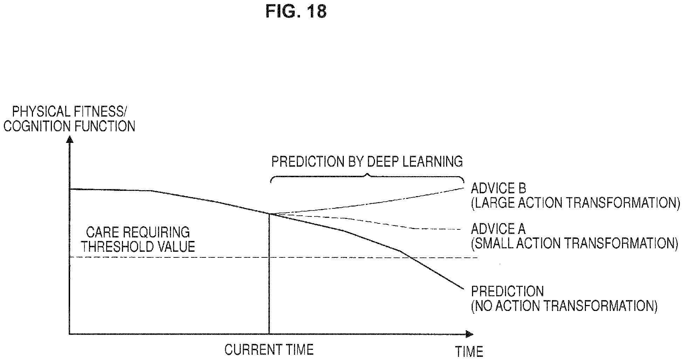

On the basis of the learning result, the prediction unit 303 performs time-series analysis of physical fitness and cognition function scores using current life information (diet, exercise, sleep, and basic information) and social participation score of the user (elderly person) as parameters, and performs prediction of future physical fitness value and cognition function value. Furthermore, from the time-series analysis result of physical fitness and cognition function scores, the prediction unit 303 can predict a possibility of the user requiring care (future necessity/nonnecessity of care in the current life situation and social participation situation, and the timing thereof). The details of care necessity prediction according to the present embodiment will be described later with reference to FIGS. 17 and 18.

In accordance with the prediction result of the prediction unit 303, the advice generation unit 304 generates an advice (personal care prevention advice) to the user that is related to a life situation or a social participation situation for care prevention. For example, the advice generation unit 304 proposes, to the user, how to improvement diet content, sleeping hours, an exercise amount, and social participation for care prevention.

### (Communication Unit 31)

The communication unit 31 connects with the network 4 in a wired or wireless manner, and performs data transmission and reception with an external device via the network 4. In addition, the communication unit 31 establishes communication connection with the network 4 by a wired/wireless local area network (LAN), Wireless Fidelity (Wi-Fi, registered trademark), or the like, for example. The communication unit 31 according to the present embodiment receives measurement results of physical fitness and a cognition function of the user from the user terminal 1, and receives life information and social participation information of the user from the nursing home DB 5. In addition, the communication unit 31 according to the present embodiment may receive life information and social participation information of the user from a wearable device, or receive information indicating a location that the user has visited, from a beacon receiving device installed at each location. In addition, the communication unit 31 transmits a care prevention advice to the user terminal 1.

### (Storage Unit 32)

The storage unit 32 is implemented by a ROM that stores programs to be used in the processing of the control unit 30, calculation parameters, and the like, and a RAM that temporarily stores appropriately varying parameters and the like. In addition, the storage unit 32 according to the present embodiment accumulates a learning result obtained by the learning unit 301, measurement results of physical fitness and a cognition function of each user, life information, a social participation score, and the like.

Hereinbefore, the configuration of the server 3 according to the present embodiment has been specifically described. Note that the configuration of the server 3 illustrated in FIG. 4 is an example, and the present embodiment is not limited to this.

### <<3. Measurement of Physical Fitness and Cognition Function>>

Subsequently, the measurement of physical fitness and a cognition function according to the present embodiment will be specifically described. In the measurement according to the present embodiment, physical fitness (muscle strength, agility, cooperativeness, reaction speed, and endurance) and a cognition function (memory and attention) of the user (elderly person) can be automatically measured safely in a short time and in a saved space in conjunction with the sensor device 2 attached to the user and the user terminal 1 (smartphone or tablet terminal).

More specifically, for example, the "muscle strength" is digitized by measuring a chair standing up and sitting down exercise and a seated leg raise duration time, the "agility" is digitized by measuring the number of steps taken in a seated position, "cooperativeness" is digitized by measuring the precision of moving feet in a triangle with keeping pace with a tempo in a seated position, the "reaction speed" is digitized by measuring a speed at which a leg is moved in a seated position in response to an instruction displayed on a screen, and the "endurance" is digitized by measuring a time for which phonation is continued in a seated position, and a decline in speed in the time of a chair standing up and sitting down exercise.

In addition, for example, the "memory" grades how many items displayed on a screen the user can memorize, and the "attention" grades how accurately and quickly the user can press a button in accordance with an instruction on a screen.

Hereinafter, execution methods of these measurement items will be specifically described.

First of all, as measurement preparation, the user attaches the sensor device 2 to a femoral region. Next, when the user performs a predetermined operation in accordance with an action instruction from the user terminal 1, the predetermined operation is detected by the sensor device 2, and measurement is automatically performed by the user terminal 1 on the basis of the detected data.

Subsequently, a specific example of each measurement item will be described. In the present embodiment, the above-described physical fitness (muscle strength, agility, cooperativeness, reaction speed, and endurance) and a cognition function (memory and attention) of the user are measured using eight measurement items, for example. Hereinafter, details of the eight measurement items will be described as examples with reference to FIGS. 5 to 13. Note that screen examples in FIGS. 5 to 13 that are to be used in the following description are examples, and the present embodiment is not limited to this.

### (1) Measurement of Phonation Duration Time

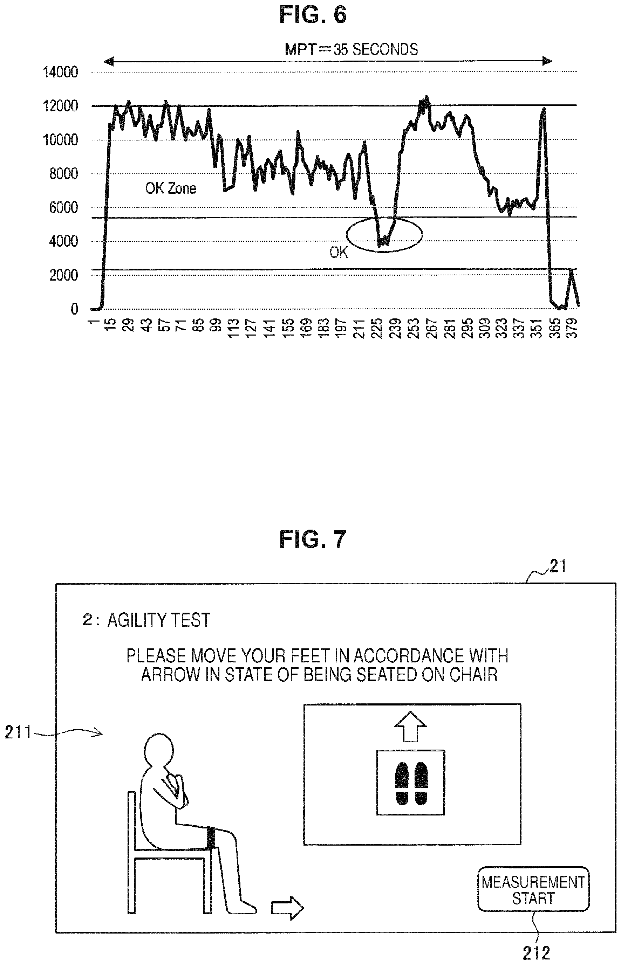

In the "measurement of a phonation duration time", a length (on a second time scale) for which voice is continued to be output is measured. As a duration time becomes longer, a better result is obtained. Here, FIG. 5 illustrates a screen display example in measuring a phonation duration time that is to be displayed on the display unit 15 of the user terminal 1. As illustrated in FIG. 5, clear indication of a measurement item "phonation duration time" and an action instruction indicating that "please output voice as long as possible" are displayed on a screen 20. After a measurement start button 201 is tapped by the user, the measurement unit 101 of the user terminal 1 collects phonation of the user by the voice input unit 14, and automatically measures a phonation duration time by measuring the level of volume of voice. As a phonation duration time, for example, as illustrated in FIG. 6, a time for which volume of voice is at certain level or more, that is to say, the maximum phonation duration time (maximum phonation time: MPT) is measured.

### (2) Agility Test

In the "agility test", an arrow is displayed at random on the display unit 15 of the user terminal 1, and a reaction speed at which the user moves his/her feet toward the direction of the arrow in a state in which the user is seated on a chair is measured. As the reaction speed is higher, a better score is obtained. Here, FIG. 7 illustrates a screen display example in the agility test that is to be displayed on the display unit 15 of the user terminal 1. As illustrated in FIG. 7, clear indication of a measurement item "agility test", an action instruction indicating that "please move your feet in accordance with an arrow in a state of being seated on a chair", and an exemplification image (still image or moving image) 211 of an arrow and an operation of moving feet are displayed on a screen 21. After a measurement start button 212 is tapped by the user, the measurement unit 101 of the user terminal 1 displays an arrow indicating a direction in which feet are to be moved, on the display unit 15, and further receives detected data of an operation of moving feet that is performed by the user, from the sensor device 2. In this manner, the user terminal 1 measures a reaction speed of the user in conjunction with the sensor device 2.

### (3) Tap Test

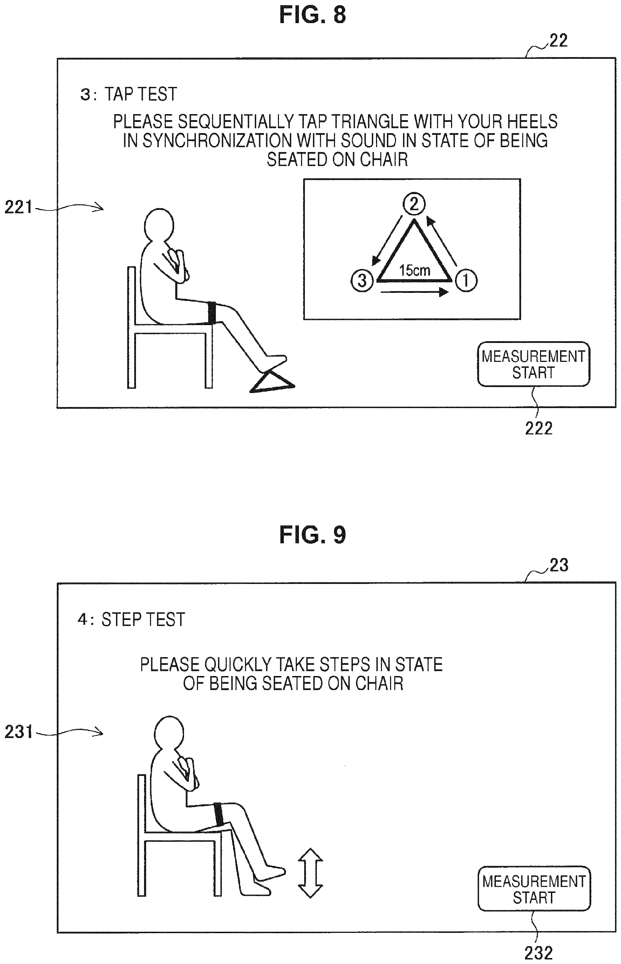

In the "tap test", precision of the user stepping on (tapping) vertices of a triangle with his/her feet with keeping pace with a tempo sound output from the user terminal 1 is measured. The number of times the tempo has been off within a predetermine time such as 30 seconds is measured, and as the number of tempo-off times is smaller, a better result is obtained (unit: point). Note that a triangle to be tapped by the user may be drawn at the user's feet (alternatively, a mat or the like on which a triangle is drawn may be laid). Here, FIG. 8 illustrates a screen display example in the tap test that is to be displayed on the display unit 15 of the user terminal 1. As illustrated in FIG. 8, clear indication of a measurement item "tap test", an action instruction indicating that "please sequentially tap a triangle with your heels in synchronization with a sound in a state of being seated on a chair", and an exemplification image (still image or moving image) 221 of an order of tapping on the triangle and a tap operation are displayed on a screen 22. After a measurement start button 222 is tapped by the user, the measurement unit 101 of the user terminal 1 outputs a tapping sound from the voice output unit 16, and further receives detected data of a tap operation performed by the user, from the sensor device 2. In this manner, the user terminal 1 measures the precision of a tap operation of the user in conjunction with the sensor device 2.

### (4) Step Test

In the "step test", the number of steps taken within a predetermined time (e.g. five seconds) in a state of being seated on a chair is measured. As the number of times is larger, a better result is obtained. Here, FIG. 9 illustrates a screen display example in the step test that is to be displayed on the display unit 15 of the user terminal 1. As illustrated in FIG. 9, clear indication of a measurement item "step test", an action instruction indicating that "please quickly take steps in a state of being seated on a chair", and an exemplification image (still image or moving image) 231 of a stepping operation are displayed on a screen 23 . When a measurement start button 232 is tapped by the user, the measurement unit 101 of the user terminal 1 measures the number of steps taken within a predetermined time such as five seconds, for example, on the basis of detected data of a step operation performed by the user that has been received from the sensor device 2. In this manner, the user terminal 1 measures the number of steps of the user in conjunction with the sensor device 2.

### (5) Leg Raise Test

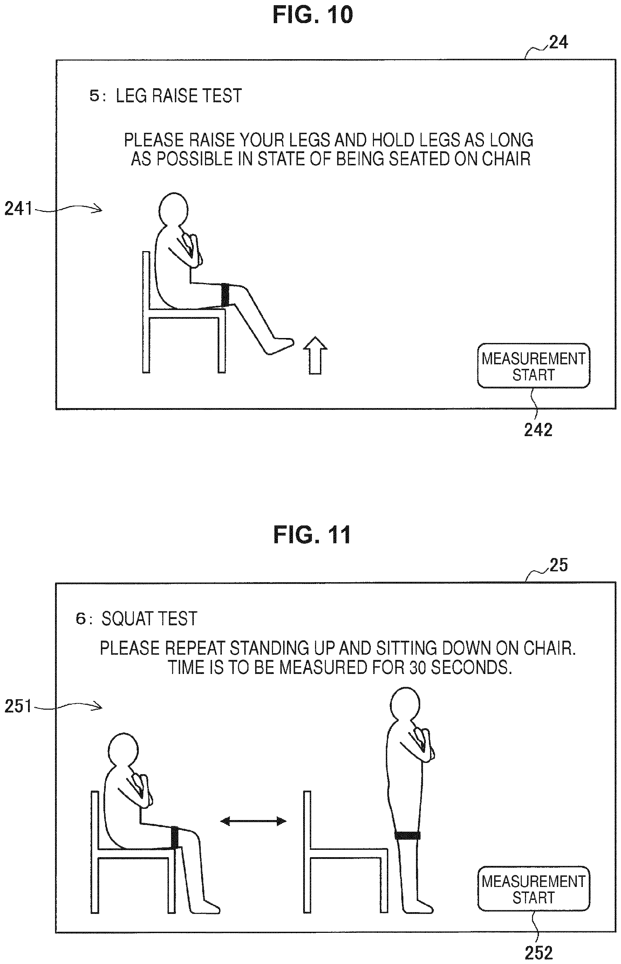

In the "leg raise test", a time (second) for which the user raises his/her legs and holds the legs in a state of being seated on a chair is measured. As a holding time becomes longer, a better result is obtained. Here, FIG. 10 illustrates a screen display example in the leg raise test that is to be displayed on the display unit 15 of the user terminal 1. As illustrated in FIG. 10, clear indication of a measurement item "leg raise test", an action instruction indicating that "please raise your legs and hold the legs as long as possible in a state of being seated on a chair", and an exemplification image (still image or moving image) 241 of a leg raising and holding operation are displayed on a screen 24. When a measurement start button 242 is tapped by the user, the measurement unit 101 of the user terminal 1 measures a holding time on the basis of detected data of the leg raising and holding operation performed by the user that has been received from the sensor device 2. In this manner, the user terminal 1 measures a leg raising and holding time of the user in conjunction with the sensor device 2.

### (6) Squat Test

In the "squat test", the number of times the user stands up from a chair and sits down within a predetermined time (e.g. 30 seconds) and a ways of motion are measured. As the number of times is larger, a better result is obtained. Here, FIG. 11 illustrates a screen display example in the squat test that is to be displayed on the display unit 15 of the user terminal 1. As illustrated in FIG. 11, clear indication of a measurement item "squat test", an action instruction indicating that "please repeat standing up and sitting down on a chair. Time is to be measured for 30 seconds", and an exemplification image (still image or moving image) 251 of a standing up and sitting down operation are displayed on a screen 25. When a measurement start button 252 is tapped by the user, the measurement unit 101 of the user terminal 1 measures the number of times the user stands up and sits down within 30 seconds, and a speed and a variation coefficient of standing up and sitting down, on the basis of detected data of the standing up and sitting down operation performed by the user that has been received from the sensor device 2.

### (7) Memory Test

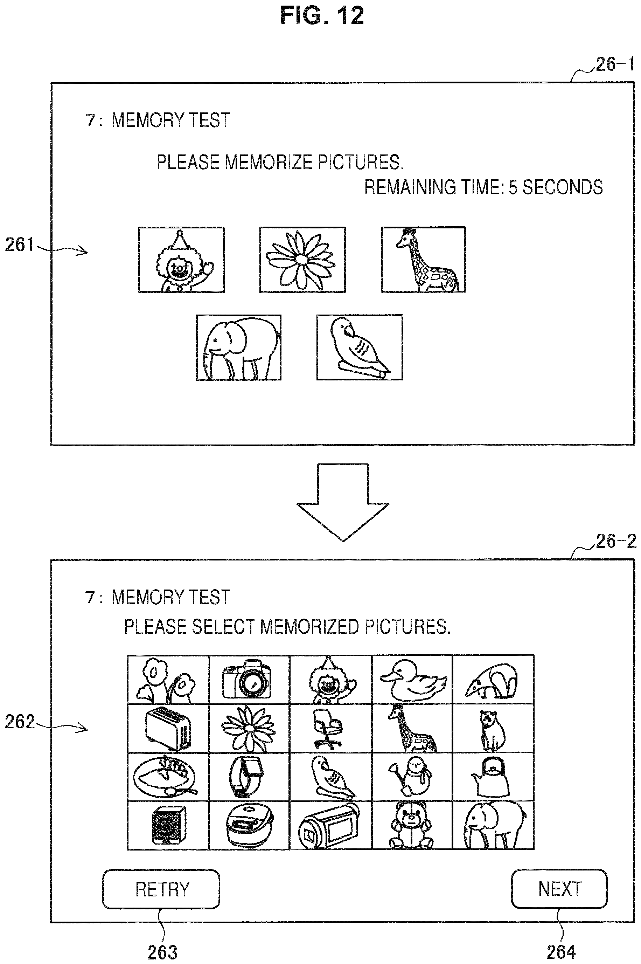

In the "memory test", for example, information such as letters and figures is displayed on the display unit 15 of the user terminal 1 for a predetermined time and the user is caused to memorize the information, and then, a number of options including the displayed information such as letters and figures are displayed and how accurately the user memorizes the information is measured. As the user memorizes a larger number of pictures, a better score is obtained. Here, FIG. 12 illustrates a screen display example in the memory test that is to be displayed on the display unit 15 of the user terminal 1. First of all, the user terminal 1 displays a screen 26-1 that displays information (a plurality of pictures in this example) 261 to be memorized by the user, as illustrated in an upper part in FIG. 12. Next, after a predetermined time elapses, as illustrated in a lower part in FIG. 12, a screen 26-2 that displays options 262 is displayed, and the user is caused to select the memorized pictures. The measurement unit 101 of the user terminal 1 determines true/false of pictures selected by the user, and performs measurement (unit: point) of memory. Note that a "retry" button 263 and a "next" button 264 are displayed on the screen 26-2, and in a case where the user desires to perform the memory test again, the user taps the "retry" button 263. In addition, in a case where the selection ends and the next test is to be performed, the user taps the "next" button 264.

### (8) Attention Test

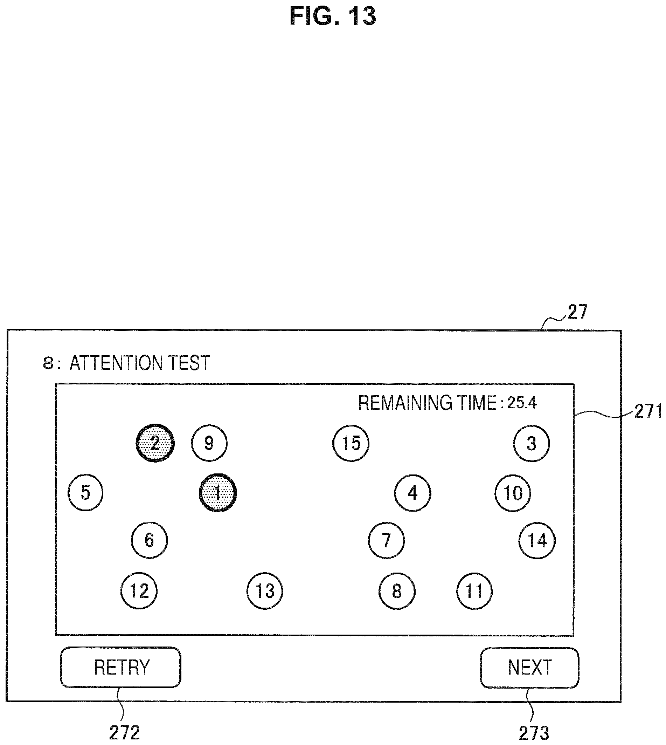

In the "attention test", for example, buttons are displayed on the display unit 15 of the user terminal 1, and how accurately and quickly the user can press a button in accordance with an instruction within a predetermined time is measured. Here, FIG. 13 illustrates a screen display example in the attention test that is to be displayed on the display unit 15 of the user terminal 1. As illustrated in FIG. 13, an image 271 in which number buttons are displayed at random is displayed on a screen 27. The user sequentially taps the number buttons as quickly and accurately as possible within a predetermined time. The measurement unit 101 of the user terminal 1 determines speed and accuracy of the user's tapping, and performs measurement (unit: point) of attention. Note that a "retry" button 272 and a "next" button 273 are displayed on the screen 27, and in a case where the user desires to perform the attention test again, the user taps the "retry" button 272. In addition, in a case where the tap manipulation ends and the next test is to be performed, the user taps the "next" button 273.

### «4. Operational Process»

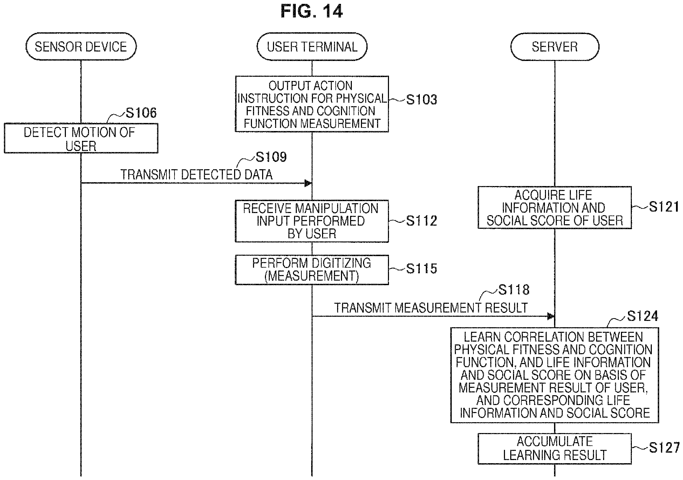

Subsequently, an operational process of the information processing system according to the present embodiment will be described with reference to FIGS. 14 to 18.

### <4-1. Learning Process>

FIG. 14 is a sequence diagram illustrating a learning process according to the present embodiment. As illustrated in FIG. 14, first of all, the user terminal 1 outputs (display-outputs or voice-outputs) an action instruction for physical fitness and cognition function measurement (step S103). Specific content of the action instruction is as described above with reference to FIGS. 5 to 13.

Next, the sensor device 2 detects a motion of the user (step S106), and transmits detected data to the user terminal 1 (step S109).

Next, the user terminal 1 receives a manipulation input performed by the user, from the manipulation input unit 13 (step S112). This is because a manipulation is sometimes directly input from the screen (touch panel) of the user terminal 1 depending on a measurement item as illustrated in FIGS. 12 to 13.

Next, the measurement unit 101 of the user terminal 1 performs physical fitness and cognition function measurement, that is to say, digitizing of muscle strength, agility, cooperativeness, reaction speed, endurance, memory, and attention on the basis of the received detected data or directly-input manipulation information (step S115).

Next, the user terminal 1 transmits a measurement result to the server 3 (step S118).

On the other hand, the server 3 acquires life information and a social score of the user (step S121). The life information of the user can be acquired from a wearable device of the user or the nursing home DB 5, for example. In addition, the social score of the user can be calculated on the basis of position information of the user or the like.

Next, the server 3 learns correlation between a measurement result of the user, and corresponding life information and social score (step S124), and accumulates a learning result (step S127).

The above-described learning process is continuously performed. In addition, in FIG. 14, only one user terminal is used as an example, but the server 3 according to the present embodiment can use measurement results (physical fitness and cognition function scores) of a number of users, and corresponding life information and social scores, and continue to learn the correlation therebetween.

### <4-2. Prediction Process>

Subsequently, a prediction process of performing time-series analysis of physical fitness and cognition function scores of the user from the current life information and social score of the user using the above-described learning result, and predicting future physical fitness value and cognition function value of the user and a possibility of future care necessity of the user will be described with reference to FIG. 15.

FIG. 15 is a flowchart illustrating a prediction process according to the present embodiment. As illustrated in FIG. 15, first of all, the social participation score calculation unit 302 of the server 3 calculates a social participation score of the user on the basis of position information of the user or the like (step S203). Here, calculation of a social participation score will be specifically described. The social participation score calculation unit 302 according to the present embodiment can calculate a social participation score by adding a point associated with a location that the user has visited, or an event that the user has participated in. For example, as illustrated in FIG. 16, in a case where points are set depending on the location, such as an own room "0 point", a dining room "3 points", and the second floor "2 points", the social participation score calculation unit 302 may calculate a social participation score by calculating a point on the basis of a location that the user has visited, and a time. For example, in a case where the user stays in his/her room for five hours, in a dining room for one hour, and stays in the second floor for two hours, because points are calculated as "5 × 0 + 1 × 3 + 2 × 2", a social participation score is calculated to be "7 points". In addition, a social participation coefficient may be further set depending on a location or an event, and a social participation score may be quantified by calculating as time × (position point × social participation coefficient).

In addition, a social participation score may be calculated by varying a coefficient depending on the number of people simultaneously existing in a specific location (e.g. a social participation score becomes higher as the user visits a location where a larger number of people assemble), or by adding a bonus point when the user participates in a specific activity or event.

In addition, as illustrated in FIG. 16, regarding position information of the user, which location the user has visited can be identified by beacon receiving devices 6a to 6c installed at the respective locations receiving a beacon transmitted from a wearable device 7 worn by the user. In addition, the identification of a location that the user has visited is not limited to this method, and the location may be determined on the basis of absolute position information measured by the wearable device 7.

In addition, the number of steps of the user may also be measured by the wearable device 7, and a score ranking of users may be displayed (e.g. displayed in real time) on a digital signage or the like in a nursing home or a specific community, together with calculated social participation scores. This produces competitive spirit, and motivation to walk or motivation of social participation can be expected to be enhanced.

Next, the server 3 acquires life information of the user (step S206). The life information of the user is acquired by the communication unit 31 from the wearable device 7 of the user or the nursing home DB 5 via the network 4, for example.

Next, on the basis of the acquired social participation score and life information, the prediction unit 303 of the server 3 utilizes the above-described learning result, and performs time-series analysis of physical fitness and cognition function scores of the user (step S209). Here, FIG. 17 illustrates an example of a time-series analysis result of physical fitness and cognition function scores. FIG. 17 illustrates physical fitness and cognition function scores predicted on the basis of a learning result from the current life situation and social participation score of May, for example. From the time-series analysis result illustrated in FIG. 17, it can be seen that there is concern that "cooperativeness" rapidly worsens from August, and in addition, "memory" rapidly worsens from October.

Next, on the basis of the time-series analysis result, the prediction unit 303 predicts future physical fitness value and cognition function value, and whether or not the user is to require care in the future (step S212). Specifically, for example, when a time-series analysis result as illustrated in FIG. 17 is reduced into one dimension as illustrated in FIG. 18, and the prediction unit 303 predicts that there is a possibility that the user is to require care, when a value falls below a predetermined care requiring threshold value. FIG. 18 is a diagram describing care necessity prediction according to the present embodiment. In the example illustrated in FIG. 18, it can be seen that there is a possibility that physical fitness and a cognition function decline to fall below a care requiring threshold value (i.e. enter a state requiring case) in the future (e.g. may be within a predetermined period).

Next, in a case where it is predicted that the user is to require care in the future (step S212/Yes), the advice generation unit 304 of the server 3 generates an advice for preventing the user from entering a state requiring case (care prevention advice) (step S215). In addition, it is also possible to generate an advice to all healthy users irrespective of whether or not a user is to require care in the future. The advice is related to a life situation and a social participation situation. A plurality of levels of advices is assumed. For example, as illustrated in FIG. 18, between a case of an advice A involving small action transformation of a life situation or the like, and a case of an advice B involving a large action transformation, a change can be generated in improvement of physical fitness and cognition function scores. A change in physical fitness and cognition function scores that is caused in accordance with an advice can also be predicted utilizing a machine learning result obtained by the learning unit 301. For example, the server 3 may present both of the advice A and the advice B, and to how much degrees care requiring risks are reduced by the respective advices may be indicated together.

Then, the control unit 30 of the server 3 presents the advice generated by the advice generation unit 304, to the user (step S218). Specifically, for example, the control unit 30 of the server 3 controls advice information to be transmitted from the communication unit 31 to the user terminal 1, and presented to the user from the user terminal 1.

As described above, in the present embodiment, by performing time-series analysis of physical fitness and a cognition function of the user utilizing a learning result, a care requiring risk (possibility of care necessity) can be predicted accurately and at an early date. In addition, when a care requiring risk is predicted, an optimum personal care prevention advice can be proposed, and care prevention can be enhanced. Note that, in the operational process described with reference to FIG. 15, an advice to the user is generated in accordance with whether or not the user is to require care in the future, but the present embodiment is not limited to this. The server 3 according to the present embodiment may generate an advice (care preventive advice) for preventing declines in physical fitness and a cognition function, to all users in accordance with a prediction result of physical fitness and a cognition function, and present the generated advice. Also in this case, similarly to the above-described example, to how much degrees declines in physical fitness and a cognition function can be suppressed depending on the extent of action transformation may be presented to the user together.

In addition, the prediction of a care requiring risk according to the present embodiment can also be utilized in an insurance product.

In addition, measurement results of physical fitness and a cognition function can also be utilized in another system by connecting thereto, such as recommendation of retail merchandises and Smart Home.

### <<5. Conclusion>>

As described above, in the information processing system according to an embodiment of the present disclosure, care prevention can be enhanced.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, a computer program for causing hardware such as a CPU, a ROM, and a RAM incorporated in the user terminal 1, the sensor device 2, the server 3, and the wearable device 7 that have been described above, to implement functions of the user terminal 1, the sensor device 2, the server 3, and the wearable device 7 can also be created. In addition, a computer-readable storage medium storing the computer program is also provided.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An information processing method including:
   acquiring, by an acquisition unit, measurement results of physical fitness measurement and a cognition function, life information, and a social participation score;
   learning, by a learning unit, correlation between a physical fitness value and a cognition function value that are based on the measurement results, and life information and a social participation score; and
   predicting, by a prediction unit, future physical fitness value and cognition function value of a user using the learned correlation on the basis of life information and a social participation score of the user.
(2) The information processing method according to (1), in which the acquisition unit calculates and acquires the social participation score on the basis of position information of a user.
(3) The information processing method according to (2), in which the acquisition unit calculates the social participation score considering a point, weighting, and a time that are set to a location, on the basis of the position information of the user.
(4) The information processing method according to (2) or (3), in which, when a signal transmitted from a transmission device worn by the user is received by a receiving device installed at a predetermined location, the acquisition unit acquires a position of the location as the position information of the user.
(5) The information processing method according to any one of (1) to (4), in which the acquisition unit calculates and acquires the social participation score on the basis of information regarding participation in a specific event.
(6) The information processing method according to any one of (1) to (5), in which the learning performs a machine learning process using the physical fitness value and the cognition function value as correct answer labels.
(7) The information processing method according to any one of (1) to (6), in which the life information includes at least any of diet information, sleep information, or exercise information.
(8) The information processing method according to (7), in which the diet information includes at least any of a diet intake timing, a calorie, or a water intake amount.
(9) The information processing method according to (7) or (8), in which the sleep information includes at least any of sleeping hours, a sleep timing, or depth of sleep.
(10) The information processing method according to any one of (7) to (9), in which the exercise information includes at least any of training, functional training, rehabilitation, the number of steps, or an amount of activity.
(11) The information processing method according to any one of (1) to (10), in which the acquisition unit acquires, as a physical fitness value, a numerical value of at least any of muscle strength, agility, cooperativeness, a reaction speed, or endurance.
(12) The information processing method according to any one of (1) to (11), in which the acquisition unit acquires, as a cognition function value, a numerical value of at least any of attention or memory.
(13) The information processing method according to any one of (1) to (12), in which the prediction unit performs time-series analysis of a physical fitness value and a cognition function value of a user using a learning result of the correlation, from current life information and social participation score of the user, and predicts future physical fitness value and cognition function value of the user.
(14) The information processing method according to any one of (1) to (13), further including:
   generating, by a generation unit, an advice related to a life situation or a social participation situation for care prevention, in accordance with a result of the prediction; and
   presenting the generated advice to the user.
(15) An information processing apparatus including:
   an acquisition unit configured to acquire measurement results of physical fitness measurement and a cognition function, life information, and a social participation score;
   a learning unit configured to learn correlation between a physical fitness value and a cognition function value that are based on the measurement results, and life information and a social participation score; and
   a prediction unit configured to predict future physical fitness value and cognition function value of a user using the learned correlation on the basis of life information and a social participation score of the user.
(16) An information processing terminal including:
   a receiving unit configured to receive detected data from a sensor device attached to a leg portion of a user; and
   a measurement unit configured to measure physical fitness of the user on the basis of the received detected data.
(17) The information processing terminal according to (16), in which the measurement unit digitizes muscle strength as measurement of the physical fitness on the basis of the detected data.
(18) The information processing terminal according to (16), in which the measurement unit digitizes agility as measurement of the physical fitness on the basis of the detected data.
(19) The information processing terminal according to (16), in which the measurement unit digitizes cooperativeness as measurement of the physical fitness on the basis of the detected data.
(20) The information processing terminal according to (16), in which the measurement unit digitizes a reaction speed as measurement of the physical fitness on the basis of the detected data.
(21) The information processing terminal according to (16), in which the measurement unit digitizes endurance as measurement of the physical fitness on the basis of the detected data.
(22) The information processing terminal according to (16), further including:
   an output unit configured to output an action instruction for instructing the user an action for measurement; and
   an input unit configured to receive an input from the user,
   in which the measurement unit measures a cognition function of the user on the basis of input data of the user that has been performed in accordance with the action instruction.
(23) The information processing terminal according to (22), in which the measurement unit digitizes attention as measurement of the cognition function on the basis of the input data.
(24) The information processing terminal according to (22), in which the measurement unit digitizes memory as measurement of the cognition function on the basis of the input data.

### Reference Signs List

- 1: user terminal
- 2: sensor device
- 3: server
- 4: network
- 5: nursing home DB
- 6: beacon receiving device
- 7: wearable device
- 10: control unit
- 101: measurement unit
- 11: network communication unit
- 12: sensor device I/F
- 13: manipulation input unit
- 14: voice input unit
- 15: display unit
- 16: voice output unit
- 17: storage unit
- 30: control unit
- 301: learning unit
- 302: social participation score calculation unit
- 303: prediction unit
- 304: advice generation unit
- 31: communication unit
- 32: storage unit

## Claims

1. An information processing method comprising:
acquiring, by an acquisition unit, measurement results of physical fitness measurement and a cognition function, life information, and a social participation score;
learning, by a learning unit, correlation between a physical fitness value and a cognition function value that are based on the measurement results, and life information and a social participation score; and
predicting, by a prediction unit, future physical fitness value and cognition function value of a user using the learned correlation on a basis of life information and a social participation score of the user.

2. The information processing method according to claim 1, wherein the acquisition unit calculates and acquires the social participation score on a basis of position information of a user.

3. The information processing method according to claim 2, wherein the acquisition unit calculates the social participation score considering a point, weighting, and a time that are set to a location, on a basis of the position information of the user.

4. The information processing method according to claim 2, wherein, when a signal transmitted from a transmission device worn by the user is received by a receiving device installed at a predetermined location, the acquisition unit acquires a position of the location as the position information of the user.

5. The information processing method according to claim 1, wherein the acquisition unit calculates and acquires the social participation score on a basis of information regarding participation in a specific event.

6. The information processing method according to claim 1, wherein the learning performs a machine learning process using the physical fitness value and the cognition function value as correct answer labels.

7. The information processing method according to claim 1, wherein the life information includes at least any of diet information, sleep information, or exercise information.

8. The information processing method according to claim 7, wherein the diet information includes at least any of a diet intake timing, a calorie, or a water intake amount.

9. The information processing method according to claim 7, wherein the sleep information includes at least any of sleeping hours, a sleep timing, or depth of sleep.

10. The information processing method according to claim 7, wherein the exercise information includes at least any of training, functional training, rehabilitation, the number of steps, or an amount of activity.

11. The information processing method according to claim 1, wherein the acquisition unit acquires, as a physical fitness value, a numerical value of at least any of muscle strength, agility, cooperativeness, a reaction speed, or endurance.

12. The information processing method according to claim 1, wherein the acquisition unit acquires, as a cognition function value, a numerical value of at least any of attention or memory.

13. The information processing method according to claim 1, wherein the prediction unit performs time-series analysis of a physical fitness value and a cognition function value of a user using a learning result of the correlation, from current life information and social participation score of the user, and predicts future physical fitness value and cognition function value of the user.

14. The information processing method according to claim 1, further comprising:
generating, by a generation unit, an advice related to a life situation or a social participation situation for care prevention, in accordance with a result of the prediction; and
presenting the generated advice to the user.

15. An information processing apparatus comprising:
an acquisition unit configured to acquire measurement results of physical fitness measurement and a cognition function, life information, and a social participation score;
a learning unit configured to learn correlation between a physical fitness value and a cognition function value that are based on the measurement results, and life information and a social participation score; and
a prediction unit configured to predict future physical fitness value and cognition function value of a user using the learned correlation on a basis of life information and a social participation score of the user.

16. An information processing terminal comprising:
a receiving unit configured to receive detected data from a sensor device attached to a leg portion of a user; and
a measurement unit configured to measure physical fitness of the user on a basis of the received detected data.

17. The information processing terminal according to claim 16, wherein the measurement unit digitizes muscle strength as measurement of the physical fitness on a basis of the detected data.

18. The information processing terminal according to claim 16, wherein the measurement unit digitizes agility as measurement of the physical fitness on a basis of the detected data.

19. The information processing terminal according to claim 16, wherein the measurement unit digitizes cooperativeness as measurement of the physical fitness on a basis of the detected data.

20. The information processing terminal according to claim 16, wherein the measurement unit digitizes a reaction speed as measurement of the physical fitness on a basis of the detected data.

21. The information processing terminal according to claim 16, wherein the measurement unit digitizes endurance as measurement of the physical fitness on a basis of the detected data.

22. The information processing terminal according to claim 16, further comprising:
an output unit configured to output an action instruction for instructing the user an action for measurement; and
an input unit configured to receive an input from the user,
wherein the measurement unit measures a cognition function of the user on a basis of input data of the user that has been performed in accordance with the action instruction.

23. The information processing terminal according to claim 22, wherein the measurement unit digitizes attention as measurement of the cognition function on a basis of the input data.

24. The information processing terminal according to claim 22, wherein the measurement unit digitizes memory as measurement of the cognition function on a basis of the input data.
